# FASCICULE DE BREVET EUROPEEN

(11) **EP 2 364 108 B1**
(45) Date de publication et mention de la délivrance du brevet: **04.09.2013**
(21) Numéro de dépôt: 09768131.6
(22) Date de dépôt: 03.11.2009
(51) Int. Cl.: A61B 5/08, A61B 5/087, A61M 16/00

(54) **SYSTEME DE DETECTION DE L'ACTIVITE MUSCULAIRE RESPIRATOIRE D'UN PATIENT SOUS ASSISTANCE RESPIRATOIRE**
SYSTEM ZUM NACHWEIS VON ATEMWEGSMUSKELAKTIVITÄT EINES PATIENTEN MIT ATEMUNTERSTÜTZUNG
SYSTEM FOR DETECTING RESPIRATORY MUSCLE ACTIVITY OF A PATIENT RECEIVING ASSISTED BREATHING

(30) Priorité: 03.11.2008 FR 0857452
(43) Date de publication de la demande: 14.09.2011
(73) Titulaire: Assistance Publique Hôpitaux De Paris, 75001 Paris (FR)
(72) Inventeur: HEYER, Laurent, F-75010 Paris (FR); BACONNIER, Pierre, F-38000 Grenoble (FR)
(74) Mandataire: Domenego, Bertrand
(86) Numéro de dépôt international: PCT/FR2009/052119
(87) Numéro de publication internationale: WO 2010/061091

(56) Documents cités:
- EP-A- 1 421 902
- US-A1- 2003 045 807
- US-A1- 2004 040 560

## Description

La présente invention concerne un système selon le préambule de la revendication 1.

Ce système s'inscrit dans le cadre de recherches sur l'optimisation des stratégies et des méthodes de surveillance et d'assistance de la fonction respiratoire en anesthésie et en réanimation.

Les progrès en anesthésie et en réanimation ont pour objectifs la réduction de la durée de la surveillance et l'amélioration de la qualité de la récupération du patient.

Une assistance respiratoire doit assurer une ventilation efficace et non délétère avec un confort acceptable pour le patient.

Dans ce contexte, l'accord entre un patient et l'appareil d'assistance respiratoire correspondant est déterminant. En situation clinique la détection d'un éventuel désaccord est essentielle pour l'optimisation de la stratégie thérapeutique.

Dans l'état de la technique, la surveillance de l'interaction entre le patient et son appareil d'assistance se heurte à la difficulté d'estimer correctement l'activité respiratoire du patient de façon robuste et non invasive.

En effet, les dispositifs non invasifs actuels sont régulièrement pris en défaut. Les limitations de ces dispositifs qui sont actuellement implémentés dans les appareils d'assistance, sont responsables d'un défaut de détection de l'activité inspiratoire du patient qui cause un désaccord entre le patient et l'appareil d'assistance et qui se traduit par une assistance sous optimale.

Les documents US 2004/0040560 A1, US 2003/0045807 A1 et EP 1 421 902 A divulguent de tels dispositifs non invasifs.

Les dispositifs alternatifs actuellement disponibles dans l'état de la technique nécessitent des capteurs de mesure de l'activité respiratoire musculaire qui sont à la fois invasifs (pression intra-thoraciques, électromyographie aiguille...) et peu robustes soit en raison des perturbations physiologiques ou de l'évolution clinique du patient soit en raison de la durée de vie du capteur pour assurer une surveillance continue.

Le but de l'invention est donc de résoudre ces problèmes.

A cet effet, l'invention a pour objet un système selon la revendication 1.

Selon d'autres aspects de l'invention, le système de détection de l'activité musculaire respiratoire d'un patient sous assistance respiratoire comprend l'une ou plusieurs des caractéristiques des revendications 2 à 10.

Ainsi, un système selon l'invention permet d'assurer une détection de l'activité musculaire respiratoire d'un patient sous assistance respiratoire, d'une part à partir de mesures non invasives et déjà disponibles la plupart du temps avec des appareils d'assistance respiratoire actuels et d'autre part avec une méthode qui permet de contourner les limitations liées à l'évolution de l'état clinique du patient, des méthodes déjà connues.

L'invention sera mieux comprise à l'aide de la description qui va suivre, donnée uniquement à titre d'exemple et faite en se référant aux dessins annexés sur lesquels :
- la figure 1 représente un schéma synoptique illustrant la structure et le fonctionnement d'un système de détection selon l'invention ;
- la figure 2 représente un schéma synoptique illustrant la structure et le fonctionnement de moyens d'estimation de pression entrant dans la constitution d'un système selon l'invention ; et
- la figure 3 illustre un cycle mécanique d'insufflation et d'exhalation.

Le système selon l'invention repose sur une détection / calcul adaptatif d'une pression musculaire représentative de l'activité musculaire respiratoire d'un patient sous assistance respiratoire.

La pression musculaire peut être détectée ou calculée à partir de signaux de débit et de pression mesurés dans le circuit pneumatique qui relie le patient à l'appareil d'assistance.

A chaque cycle mécanique, les paramètres d'un modèle mécanique du système respiratoire passif du patient sont identifiés sur des plages prédéterminées du cycle respiratoire à partir du signal de débit afin d'estimer, sur l'ensemble du cycle mécanique, la pression théorique attendue en l'absence d'activité musculaire du patient. La différence arithmétique entre cette pression théorique et la pression mesurée est représentative de la pression générée par l'activité musculaire respiratoire du patient et est appelée pression musculaire (Pmus). L'écart à zéro de cette pression indique une activité musculaire respiratoire qui est inspiratoire ou expiratoire selon le signe de cet écart. Par ce moyen, les cycles respiratoires du patient sont identifiés, un cycle respiratoire comprenant une expiration et une inspiration complètes.

Grâce à un tel système, il est possible d'adapter automatiquement les paramètres de calcul de la pression musculaire à la fois aux spécificités mécaniques du système respiratoire passif du patient et aux particularités de son comportement respiratoire de sorte que la détection de l'activité respiratoire musculaire soit en continu la plus optimale possible.

Ce système utilise les connaissances sur les conditions de déclenchement des insufflations pour adapter les paramètres du calcul de la pression musculaire, c'est-à-dire le choix du modèle de la mécanique du système respiratoire passif, la définition des zones d'identification des paramètres de ce modèle et le choix des seuils de détection d'une activité musculaire au cours du cycle mécanique.

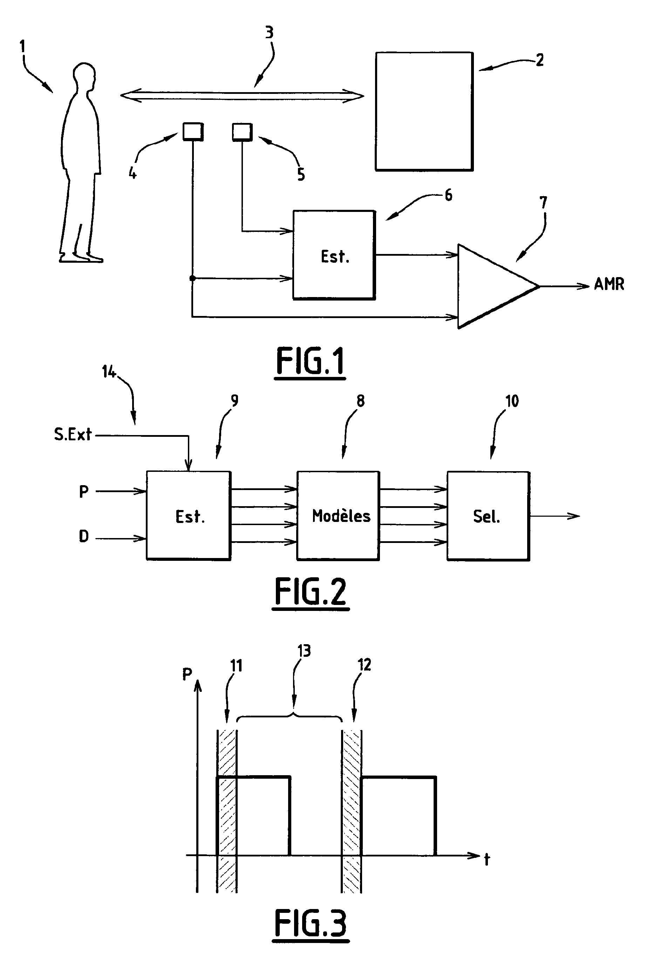

Un tel système est illustré schématiquement sur la figure 1.

Sur cette figure 1, le patient est désigné par la référence générale 1 et l'appareil d'assistance respiratoire est désigné par la référence générale 2, le patient et l'appareil respiratoire étant reliés par un circuit pneumatique désigné par la référence générale 3, de façon classique.

Dans l'exemple de réalisation illustré sur cette figure, le circuit pneumatique 3 est associé à des moyens d'acquisition de signaux de pression et de débit de l'air dans ce circuit, ces moyens étant désignés par les références 4 et 5 respectivement.

Ces moyens d'acquisition 4 et 5 sont alors adaptés pour délivrer ces signaux à des moyens d'estimation en continu de la pression théorique de l'air attendue dans le circuit pneumatique en l'absence d'activité musculaire respiratoire du patient.

Ces moyens d'estimation sont désignés par la référence générale 6 sur cette figure 1 et sont basés sur l'utilisation de moyens paramétrables et adaptatifs de modélisation du système respiratoire passif du patient comme cela sera décrit plus en détail par la suite.

Ces moyens d'estimation 6 délivrent alors une information de pression théorique estimée à des moyens de comparaison désignés par la référence générale 7, recevant sur une autre entrée la pression réellement mesurée dans le circuit pneumatique, ce qui permet de détecter en continu une différence de pression représentative d'une activité musculaire respiratoire du patient.

En fait, et comme cela a été indiqué précédemment, la différence arithmétique entre cette pression théorique estimée et la pression mesurée est représentative de la pression générée par l'activité musculaire respiratoire du patient et est appelée pression musculaire Pmus. L'écart à zéro de cette pression indique une activité musculaire respiratoire qui est inspiratoire ou expiratoire selon le signe de cet écart.

Comme cela est illustré sur la figure 2, les moyens de modélisation des moyens d'estimation comprennent un ensemble de modèles paramétrables de systèmes respiratoires passifs de patient, désigné par la référence générale 8 sur cette figure. De tels modèles sont déjà bien connus dans l'état de la technique et permettent de modéliser le comportement de la mécanique du système respiratoire du patient comme cela sera décrit plus en détail par la suite.

Ces modèles sont paramétrables, et les moyens d'estimation comprennent alors des moyens d'extraction du signal de pression mesurée, de paramètres d'entrée de ces modèles de façon à déclencher le fonctionnement de ces modèles sur la base de ces paramètres. Ces moyens d'extraction sont désignés par la référence générale 9 sur cette figure 2 et leur fonctionnement sera décrit plus en détail également par la suite.

Les moyens d'estimation 6 comprennent également des moyens de sélection du modèle le plus discriminant en terme de détection et de non détection d'activité musculaire respiratoire du patient et/ou le plus simple en terme de nombre de paramètres utilisés, pour retenir son estimation, ces moyens de sélection étant désignés par la référence générale 10.

En fait, et comme cela est illustré par exemple sur la figure 3, les moyens d'extraction 9 des paramètres sont adaptés pour extraire les paramètres d'au moins un cycle mécanique composé successivement d'une insufflation et d'une exhalation par exemple en excluant la phase de pressurisation au début du cycle mécanique courant et la phase de déclenchement de l'insufflation du cycle suivant, à la fin du cycle mécanique courant.

Ceci est illustré par les zones hachurées sur la figure 3 où l'on a représenté des cycles mécaniques successifs. La zone hachurée désignée par la référence générale 11 sur cette figure 3 correspond à la phase de pressurisation au début du cycle mécanique courant tandis que la zone hachurée désignée par la référence générale 12, correspond à la phase de déclenchement de l'insufflation du cycle suivant, à la fin du cycle mécanique courant.

L'extraction des paramètres des modèles se fait alors sur la zone désignée par la référence générale 13 entre ces deux zones d'exclusion.

Bien entendu différentes détections de ces phases sont possibles. C'est ainsi par exemple que ces phases sont détectées par des moyens d'analyse de la pression et du débit d'air dans le circuit pneumatique, les moyens d'analyse étant alors raccordés aux moyens d'acquisition des signaux de pression et de débit dans le circuit pneumatique tel que décrit précédemment.

Cependant les moyens d'analyse peuvent également être intégrés dans l'appareil d'assistance directement.

De même, les phases de pressurisation et de déclenchement de l'insufflation peuvent également être détectées par des moyens d'analyse à partir d'un signal complémentaire délivrant une information physiologique liée à l'activité musculaire respiratoire du patient tel que par exemple un signal complémentaire d'électromyogramme de surface comme désigné par la référence générale 14 sur la figure 2.

Ce système permet alors d'adapter les paramètres de calcul de la pression musculaire, c'est-à-dire de choisir le modèle de la mécanique du système respiratoire passif du patient, de définir les zones d'identification des paramètres de ce modèle et le seuil de détection d'une activité musculaire.

Il s'agit alors de sélectionner de façon adaptative, dans un jeu de différents modèles hiérarchiques et de complexité croissante, écrits sous une forme par exemple linéaire adaptée à l'identification des paramètres du modèle, un modèle adapté, par la méthode de régression linéaire multiple et au sens des moindres carrés, de façon classique.

Le modèle le plus simple, dit de référence, est une forme linéaire avec quatre paramètres P=f (V,D)=Pₒ+(Vₒ)*(V+(Rₒ+Rd*D)*D) avec V et D correspondant aux signaux de volume et de débit d'air en fonction du temps. Le volume est calculé à partir du signal D de débit par intégration en fonction du temps. Ce modèle de référence permet une détection efficace des activités inspiratoires qui déclenchent une insufflation avec une identification cycle à cycle mécanique de ses paramètres. Cette identification est réalisée à partir des signaux de débit et de volume d'air correspondant à des périodes du cycle mécanique non affectées par les phénomènes mécaniques non décrits par le modèle et non concernées par une activité musculaire inspiratoire qui déclenche une insufflation. Cette identification est efficace alors même que ces zones d'identification sont fixes d'un patient à l'autre et indépendante du comportement respiratoire du patient. Cette zone d'identification peut comporter deux parties disjointes, l'une au cours de la phase d'insufflation et l'autre au cours de la phase d'exhalation du cycle mécanique. Toujours pour ce modèle de référence, un seuil de détection d'une activité musculaire respiratoire fixe qui est compris entre 0,5 et 2 cmH20 et de préférence égal à 1 cmH20 permet une détection efficace d'une activité inspiratoire qui déclenche une insufflation.

D'autres modèles comportent n-k paramètres avec n > k et (n-k) > 4 et sont également de formes linéaires f(V,D,A) avec V,D et A qui correspondent aux signaux de volume, de débit et d'accélération de l'air en fonction du temps. L'accélération est la dérivée première du signal D de débit. Ces modèles plus complexes ont l'avantage d'avoir la capacité de décrire des phénomènes mécaniques qui ne le sont pas par le modèle de référence comme la transition entre la fin de l'insufflation et le début de l'exhalation du cycle mécanique.

Cette capacité permet de proposer une zone d'identification non plus disjointe mais continue, à la fois sur l'insufflation et l'exhalation. Cela présente deux avantages :
1) Une définition plus simple de la zone d'identification par l'exclusion d'une période à la fin d'une exhalation (ou juste avant l'insufflation) définie par un délai télé-exhalation (Dte) et d'une période au début de l'insufflation définie par un délai proto-insufflation (Dpi) ; et
2) Une amélioration de l'identification des paramètres des modèles par la prise en compte de la période de transition entre l'insufflation et l'exhalation caractérisée par des variations importantes du débit et de sa dérivée.

Néanmoins, l'augmentation de la complexité du modèle et l'extension de sa capacité à décrire des phénomènes mécaniques plus complexes induisent potentiellement une réduction de la sensibilité de la détection. Schématiquement, tout se passe comme si des phénomènes transitoires liés à une activité musculaire sont alors attribués aux caractéristiques mécaniques du système respiratoire passif.

Le système selon l'invention utilise une méthode de sélection des paramètres de calcul de la pression musculaire qui permet d'adapter le modèle et la zone d'identification de ses paramètres pour assurer une détection efficace de l'activité musculaire respiratoire sur l'ensemble du cycle mécanique.

La dégradation de la performance de l'identification de l'activité musculaire respiratoire à partir du calcul de la pression musculaire avec ces modèles plus complexes peut être efficacement compensée par une sélection appropriée du modèle mécanique et de la zone d'identification des paramètres du modèle :
- le modèle sélectionné doit être le plus proche possible de la mécanique effective du système respiratoire passif du patient ;
- la zone d'identification des paramètres doit être adaptée au comportement respiratoire du patient de telle sorte que la période du cycle mécanique exclue soit la plus proche possible de la période où le patient a une activité inspiratoire ;
- le seuil de détection de l'activité respiratoire doit être adapté en fonction de la qualité d'ajustement du modèle sélectionné et des paramètres identifiés.

Le principe de l'adaptation consiste à comparer le résultat de l'identification des activités inspiratoires qui déclenchent une insufflation pour un jeu de différents modèles et délais (Dte et Dpi) et à sélectionner les paramètres les plus adaptés sur trois critères :
1 - la capacité à assurer une détection correcte des activités connues : La détection des insufflations détectées par une activité inspiratoire (Ct-(n-k)) ou d'une activité inspiratoire qui déclenche une insufflation (Ait-(n-k)) et qui doit être identique à la détection de référence (Ct-ref).
2 - les caractéristiques d'une inspiration qui déclenche une insufflation. La zone active sélectionnée est la zone la plus courte qui précède l'insufflation et dont la durée totale (Dte + Dpi) est supérieure ou égale à la durée minimale d'une activité inspiratoire considérée comme significative (celle-ci est de l'ordre de plusieurs dixièmes de seconde et de préférence égale à 0.3 seconde).
3 - les propriétés des modèles hiérarchiquement emboîtés : le modèle sélectionné est le modèle le plus simple assurant un ajustement sur la zone d'identification des paramètres de la pression mesurée qui est statistiquement équivalent au modèle le plus complexe (ou comportant le plus de paramètres).

Pour chaque combinaison de modèles et de zones d'identification, le résultat de la détection d'une insufflation déclenchée (Ct-(n-k)) est calculé sur une période de plusieurs dizaines de cycles mécaniques (de préférence 20) et est comparé au résultat de référence (Ct-ref). Parmi les combinaisons dont le résultat est superposable au résultat de référence (Critère 1), les combinaisons correspondant à la zone d'exclusion optimale (Critère 2) sont identifiées et ensuite parmi ces combinaisons, le modèle optimal est sélectionné (Critère 3).

Cette adaptation automatique du modèle permet d'assurer d'une part une détection des activités qui déclenchent une insufflation au moins aussi efficace que la méthode de référence validée et d'autre part une détection efficace des autres activités respiratoires présentes au cours d'un cycle mécanique en cas de désadaptation de l'activité respiratoire du patient et de son appareil d'assistance.

Cette méthode présente de plus l'avantage de pouvoir suivre en continu, à la fois l'évolution du comportement respiratoire et de la mécanique du système respiratoire du patient.

Selon un premier mode de réalisation, le dispositif utilise la détection des insufflations déclenchées par l'inspiration du patient par le modèle de référence avec quatre paramètres. Cette réalisation présente l'avantage de réduire les signaux d'entrée du dispositif aux seuls signaux de pression et de débit.

Selon un deuxième mode de réalisation, l'information sur le mécanisme de déclenchement des insufflations, est fournie par un signal additionnel qui peut être fourni par l'appareil d'assistance ou par un capteur additionnel. Dans le premier cas, il s'agit d'un signal représentatif de l'état d'ouverture et de déclenchement des valves d'insufflation internes à l'appareil d'assistance. Dans le deuxième cas, il s'agit d'un signal représentatif de l'activité inspiratoire du patient fourni par un capteur non-invasif et distinct des signaux de pression ou de débit comme un détecteur d'activité d'un muscle avec une activité inspiratoire à partir d'un électromyogramme de surface (sEMG ou sMMG) ou encore de mouvement (impédencemétrie).

Ce système automatique de détection a été testé expérimentalement dans trois situations :
- i) pour évaluer la méthode de sélection du modèle le plus simple adapté (Critère 3) ;
- ii) pour évaluer la méthode de définition optimale des zones d'identification (Critère 2) ;
- iii) pour évaluer la combinaison de ces méthodes.

### 1) Evaluation de la méthode de sélection du modèle le plus simple adapté (critère 3) :

Cette étude a été réalisée à partir d'enregistrements de pression et de débit réalisés sur un poumon mécanique artificiel ventilé par un appareil d'assistance dont les caractéristiques mécaniques sont parfaitement connues. La méthode de sélection sur la qualité de l'ajustement mesurée par la comparaison statistique du résidu conduit à sélectionner comme modèle le plus simple statistiquement équivalent (avec un risque alpha de 1% ou 5%) le modèle minimum nécessaire pour décrire la mécanique du poumon mécanique.

### 2) Evaluation de la méthode de définition optimale des zones d'identification (critère 2) :

Cette étude a été réalisée à partir d'enregistrements anciens réalisés chez 14 patients sous assistance respiratoire partielle qui avaient subi un échec de sevrage bien conduit et pour cela redevables d'une exploration spécifique et invasive de leur activité respiratoire par la mesure de la pression oesophagienne.

Chez ces patients, la capacité de détection par la méthode non invasive est mesurée par la concordance entre l'activité détectée par le calcul de la pression musculaire et l'activité détectée par la lecture de la pression oesophagienne. La valeur de la concordance calculée avec la méthode automatique de sélection des zones d'identification est comparée par la méthode de Bland et Altman à la concordance optimale observée pour l'ensemble des combinaisons possibles de délais qui définissent la zone d'identification et pour un modèle complexe fixe. L'analyse de la représentation graphique permet de dire que les deux méthodes sont interchangeables avec une réduction de la concordance avec la méthode automatique faible de 4% et un écart moyen de 4%.

Chez ces patients, les valeurs de délais optimaux de définition de la zone d'exclusion ont été identifiées pour la détection des activités qui déclenchent une insufflation et pour une détection de l'ensemble des activités inspiratoires déclenchantes ou non. Ces délais optimaux et en particulier le délai télé-exhalation (Dte) sont directement liés aux délais mesurés entre le début d'activité inspiratoire et le déclenchement de l'insufflation défini à partir de la pression oesophagienne.

### 3) Evaluation de la méthode de définition optimale :

Cette étude a été réalisée à partir d'enregistrements anciens réalisés chez 17 patients sous assistance respiratoire partielle redevables d'une exploration non-invasive de leur activité respiratoire par la mesure d'un électromyogramme de surface du diaphragme (sEMG). La détection des activités inspiratoires déclenchantes et non par la méthode automatique est comparée par la méthode de Bland et Altman à celle fournie par la lecture manuelle des signaux sEMG, Débit et Pression. Ces deux méthodes sont superposables pour la détection des deux types d'activités inspiratoires qui déclenchent et qui ne déclenchent pas une insufflation.

Il est bien entendu que différents modes de réalisation d'un tel système peuvent encore être envisagés.

## Revendications

1. Système de détection de l'activité musculaire respiratoire d'un patient (1) sous assistance respiratoire, raccordé à un appareil d'assistance (2) par un circuit pneumatique (3) comportant des moyens (4, 5) d'acquisition de signaux de pression et de débit de l'air dans le circuit pneumatique (3),
**caractérisé en ce que** lesdits moyens (4, 5) d'acquisition de signaux de pression et de débit de l'air dans le circuit pneumatique (3) sont à destination de moyens (6) d'estimation en continu de la pression théorique de l'air attendue dans le circuit pneumatique en l'absence d'activité musculaire respiratoire du patient, et **en ce qu'**il comporte des moyens (7) de comparaison des pressions théoriques estimées et mesurées pour détecter en continu une différence de pression représentative d'une activité musculaire respiratoire du patient.

2. Système de détection de l'activité musculaire respiratoire d'un patient sous assistance respiratoire selon la revendication 1, **caractérisé en ce que** les moyens (6) d'estimation comprennent des moyens (8) paramétrables et adaptatifs de modélisation du système respiratoire passif du patient.

3. Système de détection de l'activité musculaire respiratoire d'un patient sous assistance respiratoire selon la revendication 2, **caractérisé en ce que** les moyens (8) de modélisation se présentent sous la forme de modèles fonction au moins du volume et du débit d'air circulant dans le circuit pneumatique.

4. Système de détection de l'activité musculaire respiratoire d'un patient sous assistance respiratoire selon la revendication 2 ou 3, **caractérisé en ce que** les moyens de modélisation comprennent un ensemble (8) de modèles paramétrables et **en ce que** les moyens d'estimation comprennent des moyens (9) d'extraction du signal de pression mesurée, de paramètres d'entrée de ces modèles, de façon à déclencher le fonctionnement de ces modèles sur la base de ces paramètres et des moyens (10) de sélection du modèle le plus discriminant en terme de détection et de non-détection d'activité musculaire respiratoire du patient et/ou le plus simple en terme de nombre de paramètres utilisés, pour retenir son estimation.

5. Système de détection de l'activité musculaire respiratoire d'un patient sous assistance respiratoire selon la revendication 4, **caractérisé en ce que** les moyens (9) d'extraction des paramètres sont adaptés pour extraire les paramètres sur au moins un cycle mécanique composé successivement d'une insufflation et d'une exhalation, en excluant la phase (11) de pressurisation au début du cycle mécanique courant et la phase (12) de déclenchement de l'insufflation du cycle suivant, à la fin du cycle mécanique courant.

6. Système de détection de l'activité musculaire respiratoire d'un patient sous assistance respiratoire selon la revendication 5, **caractérisé en ce que** la phase (11) de pressurisation et la phase (12) de déclenchement de l'insufflation sont détectées par des moyens d'analyse de la pression et du débit d'air dans le circuit pneumatique (3).

7. Système de détection de l'activité musculaire respiratoire d'un patient sous assistance respiratoire selon la revendication 6, **caractérisé en ce que** les moyens d'analyse sont raccordés aux moyens (4, 5) d'acquisition des signaux de pression et de débit dans le circuit pneumatique (3).

8. Système de détection de l'activité musculaire respiratoire d'un patient sous assistance respiratoire selon la revendication 6 ou 7, **caractérisé en ce que** les moyens d'analyse sont intégrés dans l'appareil d'assistance (2).

9. Système de détection de l'activité musculaire respiratoire d'un patient sous assistance respiratoire selon la revendication 5, **caractérisé en ce que** les phases (11) de pressurisation et (12) de déclenchement de l'insufflation sont détectées par des moyens d'analyse à partir d'un signal complémentaire (14) délivrant une information physiologique liée à l'activité musculaire respiratoire du patient.

10. Système de détection de l'activité musculaire respiratoire d'un patient sous assistance respiratoire selon la revendication 9, **caractérisé en ce que** le signal complémentaire est un signal d'électromyogramme de surface.

## Patentansprüche

1. System zur Detektion der Atmungsmuskulatur-Aktivität eines Patienten (1) unter Atmungsunterstützung, verbunden mit einem Unterstützungsapparat (2) über einen pneumatischen Kreis (3), der Mittel (4, 5) zur Erfassung von Signalen des Drucks und des Durchflusses der Luft in dem pneumatischen Kreis (3) aufweist,
**dadurch gekennzeichnet, dass** die besagten Mittel (4, 5) zur Erfassung von Signalen des Drucks und des Durchflusses der Luft in dem pneumatischen Kreis (3) Mitteln (6) zur kontinuierlichen Abschätzung des theoretischen Luftdrucks zugeordnet sind, der im pneumatischen Kreis (3) erwartet wird in Abwesenheit von Atmungsmuskulatur-Aktivität des Patienten, und dass es Mittel (7) zum Vergleichen der theoretischen abgeschätzten und der gemessenen Drücke aufweist zum kontinuierlichen Detektieren einer Druckdifferenz, die für eine Atmungsmuskulatur-Aktivität des Patienten repräsentativ ist.

2. System zur Detektion der Atmungsmuskulatur-Aktivität eines Patienten unter Atmungsunterstützung gemäß Anspruch 1, **dadurch gekennzeichnet, dass** die Mittel (6) zur Abschätzung aufweisen Mittel (8), die parametrierbar und adaptiv sind zur Modellierung des passiven Atmungssystems des Patienten.

3. System zur Detektion der Atmungsmuskulatur-Aktivität eines Patienten unter Atmungsunterstützung gemäß Anspruch 2, **dadurch gekennzeichnet, dass** die Mittel (8) zur Modellierung in Form von Modellen vorliegen abhängig zumindest vom Volumen und vom Durchfluss der Luft, die in dem pneumatischen Kreis zirkuliert.

4. System zur Detektion der Atmungsmuskulatur-Aktivität eines Patienten unter Atmungsunterstützung gemäß Anspruch 2 oder 3, **dadurch gekennzeichnet, dass** die Mittel zur Modellierung aufweisen eine Gruppe (8) von parametrierbaren Modellen, und dass die Mittel zur Abschätzung aufweisen Mittel (9) zum Extrahieren von Eingangsparametern dieser Modelle aus dem gemessenen Drucksignal, um die Funktion dieser Modelle auf Basis dieser Parameter zu bewirken, und Mittel (10) zur Auswahl des Modells, das am meisten unterscheidend ist bezüglich der Detektion und der Nicht-Detektion von Atmungsmuskulatur-Aktivität des Patienten und/oder das das einfachste ist bezüglich der Anzahl an verwendeten Parametern, zum Berücksichtigen dessen Abschätzung.

5. System zur Detektion von Atmungsmuskulatur-Aktivität eines Patienten unter Atmungsunterstützung gemäß Anspruch 4, **dadurch gekennzeichnet, dass** die Mittel (9) zur Extraktion der Parameter angepasst sind zum Extrahieren der Parameter über wenigstens einen mechanischen Zyklus, der sukzessive zusammengesetzt ist aus einer Einatmung und einer Ausatmung, unter Ausschließen der Phase (11) des Druckausgleichs am Anfang des mechanischen Zyklus und der Phase (12) des Beginns der Einatmung des nachfolgenden Zyklus am Ende des momentanen mechanischen Zyklus.

6. System zur Detektion von Atmungsmuskulatur-Aktivität eines Patienten unter Atmungsunterstützung gemäß Anspruch 5, **dadurch gekennzeichnet, dass** die Phase (11) des Druckausgleichs und die Phase (12) des Beginns der Einatmung detektiert werden mittels Mitteln zur Analyse des Drucks und des Durchflusses der Luft in dem pneumatischen Kreis (3).

7. System zur Detektion der Atmungsmuskulatur-Aktivität eines Patienten unter Atmungsunterstützung gemäß Anspruch 6, **dadurch gekennzeichnet, dass** die Mittel zur Analyse verbunden sind mit Mitteln (4, 5) zur Erlangung der Signale des Drucks und des Durchflusses in dem pneumatischen Kreis (3).

8. System zur Detektion der Atmungsmuskulatur-Aktivität eines Patienten unter Atmungsunterstützung gemäß Anspruch 6 oder 7, **dadurch gekennzeichnet, dass** die Mittel zur Analyse in dem Unterstützungsapparat (2) integriert sind.

9. System zur Detektion der Atmungsmuskulatur-Aktivität eines Patienten unter Atmungsunterstützung gemäß Anspruch 5, **dadurch gekennzeichnet, dass** die Phasen (11) des Druckausgleichs und (12) des Beginns der Einatmung detektiert werden mittels der Mittel zur Analyse ausgehend von einem komplementären Signal (14), das eine physiologische Information liefert, die mit der Atmungsmuskulatur-Aktivität des Patentien zusammenhängt.

10. System zur Detektion der Atmungsmuskulatur-Aktivität eines Patienten unter Atmungsunterstützung gemäß Anspruch 9, **dadurch gekennzeichnet, dass** das komplementäre Signal ein Flächen-Elektromyogramm-Signal ist.

## Claims

1. A system for detecting respiratory muscle activity of a patient (1) receiving assisted breathing, which is connected to an assistance apparatus (2) by a pneumatic circuit (3) comprising means (4, 5) for acquiring air pressure and flow signals in the pneumatic circuit (3),
**characterized in that** the means (4, 5) for acquiring air pressure and flow signals in the pneumatic circuit (3) are directed to means (6) for continuously estimating the theoretical air pressure expected in the pneumatic circuit in absence of respiratory muscle activity from the patient, and **in that** it comprises means (7) for comparing estimated and actual theoretical pressures in order to continuously detect a pressure differential showing respiratory muscle activity in the patient.

2. The system for detecting respiratory muscle activity of a patient receiving assisted breathing according to claim 1, **characterized in that** the estimating means (6) comprises configurable and adaptive means (8) for modeling the patient's passive respiratory system.

3. The system for detecting respiratory muscle activity of a patient receiving assisted breathing according to claim 2, **characterized in that** the modeling means (8) assumes the form of models depending at least on the air volume and flow circulating in the pneumatic circuit.

4. The system for detecting respiratory muscle activity of a patient receiving assisted breathing according to claim 2 or 3, **characterized in that** the modeling means comprises a set (8) of configurable models and **in that** the estimating means comprises a means (9) for extracting, from the measured pressure signal, input parameters for said models, so as to trigger the operation of these models based on these parameters, and a means (10) for selecting the most discriminating model in terms of detection and non-detection of respiratory muscle activity by the patient and/or the simplest in terms of number of parameters used, to make its estimate.

5. The system for detecting respiratory muscle activity of a patient receiving assisted breathing according to claim 4, **characterized in that** the means (9) for extracting parameters is adapted to extract the parameters over at least one mechanical cycle made up successively of an inflation and an exhale, excluding the pressurization phase (11) at the beginning of the mechanical cycle in progress and the phase (12) triggering the inflation for the following cycle, at the end of the mechanical cycle in progress.

6. The system for detecting respiratory muscle activity of a patient receiving assisted breathing according to claim 5, **characterized in that** the pressurization phase (11) and the phase (12) triggering the inflation are detected by a means for analyzing the air pressure and flow in the pneumatic circuit (3).

7. The system for detecting respiratory muscle activity of a patient receiving assisted breathing according to claim 6, **characterized in that** the analysis means is connected to the means (4, 5) for acquiring pressure and flow signals in the pneumatic circuit (3).

8. The system for detecting respiratory muscle activity of a patient receiving assisted breathing according to claim 6 or 7, **characterized in that** the analysis means is integrated into the assistance apparatus (2).

9. The system for detecting respiratory muscle activity of a patient receiving assisted breathing according to claim 5, **characterized in that** the pressurization (11) and inflation triggering (12) phases are detected by an analysis means from a complementary signal (14) delivering physiological information related to the patient's respiratory muscle activity.

10. The system for detecting respiratory muscle activity of a patient receiving assisted breathing according to claim 9, **characterized in that** the complementary signal is a surface electromyogram signal.
